# EUROPEAN PATENT SPECIFICATION

(11) **EP 0 996 363 B1**
(45) Date of publication and mention of the grant of the patent: **27.10.2004**
(21) Application number: 98933199.6
(22) Date of filing: 02.07.1998
(51) Int. Cl.: A61B 8/00, A61B 17/42, A61B 19/00, A61B 8/12

(54) **ENDOVAGINAL SONOGRAPHY GUIDANCE OF INTRA-UTERINE PROCEDURES**
LEITVORRICHTUNG FÜR ENDOVAGINALE ECHOGRAPHIE ZUR ANWENDUNG BEI INTRAUTERINEN EINGRIFFEN
GUIDAGE D'ECHOGRAPHIE ENDOVAGINALE LORS D'INTERVENTIONS INTRA-UTERINES

(30) Priority: 17.07.1997 US 896052
(43) Date of publication of application: 03.05.2000
(73) Proprietor: Ron-Tech Medical Ltd., 46407 Herzlia (IL)
(72) Inventor: TEPPER, Ron, 46407 Herzlia (IL); HADANI, Ron, Teaneck, NJ 07666 (US)
(74) Representative: Lampe, Sigmar
(86) International application number: PCT/US1998/014012
(87) International publication number: WO 1999/003399

(56) References cited:
- US-A- 2 482 622
- US-A- 3 709 215
- US-A- 4 742 829
- US-A- 4 838 506
- US-A- 5 037 430
- US-A- 5 090 414
- US-A- 5 199 437

## Description

### FIELD AND BACKGROUND OF THE INVENTION

The present invention relates to apparatus system for real-time endovaginal sonography guidance of intra-uterine, cervical and tubal procedures.

Endovaginal ultrasound transducers for diagnosis and monitoring of obstetric and gynaecologic disorders are well known in the art, for example from US 5,199,437. However, the use of such endovaginal probes for real-time monitoring of surgical procedures is very limited.

Examples of endovaginal ultrasound transducers for real-time monitoring and guidance of surgical procedures are disclosed in U.S. Pat. Nos. 4,497,325, 4,671,292, 4,681,103, 4,742,829, 4,877,033, 4,883,059 and 5,280,427.

Most of these disclosures provide ultrasound transducers including a needle and/or catheter guide attached thereto for introducing a needle and/or catheter to a targeted tissue. However, the surgical procedures which may be carried out by such endovaginal probes are usually very limited and include puncturing and drainage of abscesses, local tissue sampling and fluid collection.

United States Patent No. 5,037,430 to Hasson discloses a clamping device for positioning and holding gynaecological instruments. A second clamp is located intermediate the ends of the clamping device for releasably clamping onto the gynaecological instrument to hold the instrument in proper position relative to the uterus.

Devices for engaging endovaginal ultrasound transducers are known as well. For example, United States Patent Number 4,838,506 to Cooper discloses a holder for a needle guide sleeve for use in cooperation with an ultrasonic probe. The holder has an arm with a slot through which the needle guide sleeve rests and is movable in two directions.

The prior art fails to provide endovaginal apparatus for real-time monitoring and guidance of more complicated surgical procedures. In particular, the prior art fails to provide endovaginal apparatus for real-time monitoring and guidance of intra-uterine, cervical and tubal procedures requiring manual dexterity of a surgeon, such as, but not limited to, (i) curettage or evacuation of the uterine cavity for diagnostic and/or therapeutic purposes; (ii) removal of an endometrial polyp, submucous myoma or other tissue; (iii) introduction or extraction of an intra-uterine contraceptive device (IUCD) and other foreign bodies; (iv) systematic sampling of the endometrium and/or the endocervix for diagnostic purposes; (v) embryo transfer into the endometrial cavity; (vi) embryo transfer into the fallopian tube; (vii) fallopian tube canullation; (viii) ultrasound guided fetal reduction; (ix) simultaneous insertion of an image transmitting device such as endoscopy equipment into the uterine cavity for complementary diagnostic and/or therapeutic purposes; (x) chorionic villi sampling; (xi) fetoscopy; (xii) amniocenthesis; and (xiii) fetal tissue sampling.

Transabdominal ultrasound is regularly not used for real-time monitoring and guidance of such surgical procedures due to its relatively limited resolution, the need to keep the patient's urinary bladder full during operation, and the need of extra-operating stuff.

As a consequence, such surgical procedures are in many cases carried out blindly, relying solely on the "feel" transmitted through manual manipulation of the instrument and the surgeon's experience. However, when the position or size of the uterus is incorrectly diagnosed by the surgeon, uterine perforation may occur with remarkable ease. The chances of perforation are higher in the presence of cervical stenosis or uterine malignancy (endometrial or sarcoma).

The main dangers of such uterine perforation include bleeding and trauma to the abdominal viscera as well as damage to internal organs such as bowel, omentum, mesentery, ureter and fallopian tube. Thus, exploration of the abdominal cavity by laparoscopy or laparotomy is often needed due to accidental uterine perforation. Other poor outcomes of blind operation include, for example, failure to completely remove uterine tissues such as placental or fetal tissues, which necessitates a second curettage under general anesthesia, or misplacement of foreign bodies or embryos therein.

There is thus a widely recognized need for, and it would be highly advantageous to have, an apparatus for real-time endovaginal sonography guidance and monitoring of intra-uterine, cervical and tubal surgical and non-surgical procedures.

Specifically, it would be advantageous to have such apparatus for real time monitoring and guidance of procedures such as, but not limited to, (i) curettage or evacuation of the uterine cavity for diagnostic and/or therapeutic purposes; (ii) removal of an endometrial polyp, submucous myoma or other tissue; (iii) introduction or extraction of an intra-uterine contraceptive device (IUCD) and other foreign bodies; (iv) systematic sampling of the endometrium and/or the endocervix for diagnostic purposes; (v) embryo transfer into the endometrial cavity; (vi) embryo transfer into the fallopian tube; (vii) fallopian tube canullation; (viii) ultrasound guided fetal reduction; (ix) simultaneous insertion of an image transmitting device such as endoscopy equipment into the uterine cavity for complementary diagnostic and/or therapeutic purposes; (x) chorionic villi sampling; (xi) fetoscopy; (xii) amniocentesis; and (xiii) fetal tissue sampling.

It would be further advantageous to have such apparatus which enables the surgeon to perform such intra-uterine and cervical procedures safely, conveniently and efficiently. In particular, it would be advantageous to have such apparatus so as to substantially shorten the duration of surgical procedures currently carried out under general anaesthesia and to reduce the rate of complications associated with such procedures.

It would be further advantageous to have apparatus which enables the surgeon to simultaneously monitor and guide surgical and non-surgical intra-uterine procedures by means of endovaginal sonography and/or intra-uterine endoscopy.

### SUMMARY OF THE INVENTION

According to a first aspect of the present invention, there is provided an apparatus for guidance and monitoring of intra-uterine, cervical and tubal procedures, comprising an assembly including: (a) an endovaginal ultrasound transducer being adapted for insertion into a portion of a patient's vagina; characterised in that the assembly further includes:(b) a cervical holder, including: (i) two arms having a securing member; and (ii) two holders, said holders being for holding the patient's cervix; and (c) a connector for interconnecting said ultrasound transducer and said cervical holder.

According to a second aspect of the present invention there is further provided a system for guidance and monitoring of intra-uterine, cervical and tubal procedures, the system comprising (a) an assembly being operable by a weak hand of a surgeon, the assembly being provided by an apparatus according to the first aspect of the present invention; (b) a medical instrument for performing the procedure, the medical instrument being operable by a strong hand of the surgeon; and (c) a device for monitoring an alignment of the medical instrument with respect to the endovaginal ultrasound transducer and therefore also with respect to the ultrasound beam.

The present invention successfully addresses the shortcomings of the presently known configurations by providing an apparatus and system for real-time endovaginal sonography guidance and monitoring of intra-uterine, cervical and tubal procedures, such as, but not limited to, (i) curettage or evacuation of the uterine cavity for diagnostic and/or therapeutic purposes; (ii) removal of an endometrial polyp, submucous myoma or other tissue; (iii) introduction or extraction of an intra-uterine contraceptive device (IUCD) and other foreign bodies; (iv) systematic sampling of the endometrium and/or the endocervix for diagnostic purposes; (v) embryo transfer into the endometrial cavity; (vi) embryo transfer into the fallopian tube; (vii) fallopian tube canullation; (viii) ultrasound guided fetal reduction; (ix) simultaneous insertion of an image transmitting device such as endoscopy equipment into the uterine cavity for complementary diagnostic and/or therapeutic purposes; (x) chorionic villi sampling; (xi) fetoscopy; (xii) amniocentesis; and (xiii) fetal tissue sampling.

The present invention discloses novel apparatus and system for real-time endovaginal sonography guidance and monitoring of intra-uterine, cervical and tubal procedures. When using an apparatus or system according to the present invention, the cervical holder and the endovaginal ultrasound transducer are preferably held by the weak of the surgeon so that the other strong hand of the surgeon is free to conduct the surgical procedure. Since in most cases the diameter of the endovaginal ultrasound transducer is substantially small, the surgeon may conveniently introduce a medical instrument such as a curette through the cervix into the uterine cavity of the patient. Thus, the surgical procedure is continuously guided and monitored by means of the endovaginal ultrasound transducer. The medical instrument (or tool) is aligned with respect to the ultrasonic beam of the transducer, such that the surgeon can, view the treated region before, during and after the procedure, and conveniently and safely direct the medical instrument of choice to that region.

### BRIEF DESCRIPTION OF THE DRAWINGS

The invention is herein described, by way of example only, with reference to the accompanying drawings, wherein: .
FIG. 1 is a schematic illustration of an apparatus according to the present invention;
FIG. 2 is a detailed description of a preferred embodiment of a connector according to the present invention;
FIG. 3 illustrates the use of the apparatus shown in Figure 1 for guiding and monitoring intra-uterine curettage;
FIGs. 4-7 are schematic illustrations of alternative embodiments of a system according to the present invention including the apparatus shown in Figure 1, a medical instrument and a device for monitoring the alignment of the medical instrument with respect to the ultrasound transducer and therefore also with respect to the ultrasound beam; and
FIG. 8 is a schematic illustration of a screen displaying images according to the present invention.

### DESCRIPTION OF THE PREFERRED EMBODIMENTS

The present invention is of apparatus and system for real-time endovaginal sonography guidance of intra-uterine, cervical and tubal surgical and non-surgical procedures. Specifically, the present invention can be used to guide and monitor intra uterine, cervical and tubal procedures such as, but not limited to, (i) curettage or evacuation of the uterine cavity for diagnostic and/or therapeutic purposes; (ii) removal of an endometrial polyp, submucous myoma or other tissue; (iii) introduction or extraction of an intra-uterine contraceptive device (IUCD) and other foreign bodies; (iv) systematic sampling of the endometrium and/or the endocervix for diagnostic purposes; (v) embryo transfer into the endometrial cavity; (vi) embryo transfer into the fallopian tube; (vii) fallopian tube canullation; (viii) ultrasound guided fetal reduction; and (ix) simultaneous insertion of an image transmitting device such as endoscopy equipment into the uterine cavity for complementary diagnostic and/or therapeutic purposes.

The principles and operation of apparatus and system according to the present invention may be better understood with reference to the drawings and the accompanying description.

Before explaining at least one embodiment of the invention in detail, it is to be understood that the invention is not limited in its application to the details of construction and the arrangement of the components set forth in the following description or illustrated in the drawings. The invention is capable of other embodiments or of being practiced or carried out in various ways. Also, it is to be understood that the phraseology and terminology employed herein is for the purpose of description and should not be regarded as limiting.

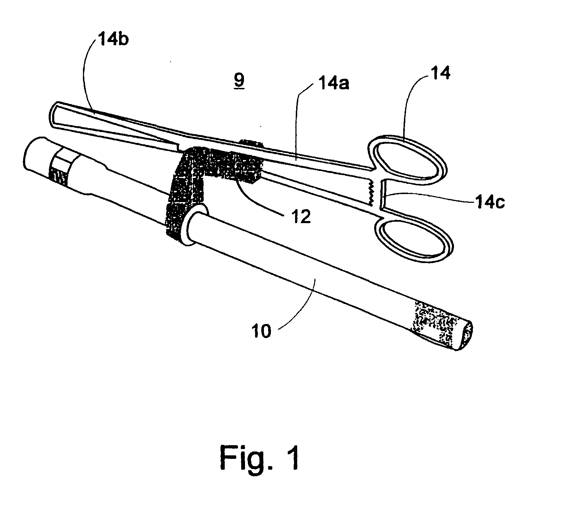

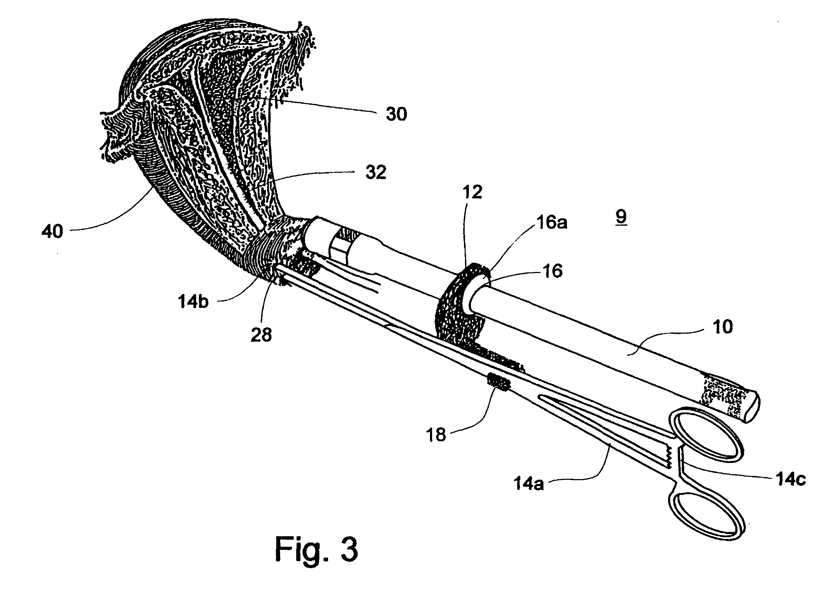

Referring now to the drawings, Figure 1 illustrates a preferred embodiment of an apparatus according to the present invention, which is referred to herein below as apparatus **9**. As shown, apparatus **9** includes an endovaginal ultrasound transducer **10**, a cervical holder **14** and a connector **12** for connecting endovaginal ultrasound transducer **10** to cervical holder **14**. Preferably, endovaginal transducer **10** features substantially small diameter so as to allow simultaneous insertion of transducer **10** and cervical holder **14** into the patient's vagina. Cervical holder **14** is preferably a conventional cervical holder, including two arms **14a** each including a securing member **14c;** and two holders **14b** for holding a cervix of a patient, as for example shown in Figure 3.

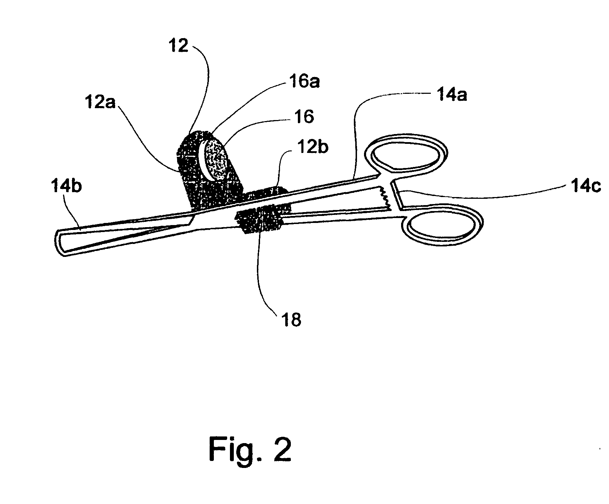

As shown in Figure 2, connector **12** preferably includes a first segment **12a** and a second segment **12b**. Preferably, first segment **12a** features a flat configuration and includes a circular aperture **16** for accommodating transducer **10** therein. Preferably, an adjustment annular member **16a** is embedded within aperture **16** for adjusting the orientation of transducer **10** relative to first segment **12a**. Preferably, second segment **12b** features an elongated configuration and includes a protrusion **18** for locking connector **12** between arms **14a,** as holders **14b** grip the cervix of the patient upon securing of cervical holder **14** by means of securing member **14c**. The dimensions of connector **12** may be specifically adapted for various probes. Connector **12** may be made of any appropriate material. Preferably, connector **12** is disposable.

According to an alternative configuration, connector **12** and cervical holder **14** are integrally made. Alternatively, connector **12** and endovaginal ultrasound transducer **10** are integrally made.

Figure 3 illustrates the use of apparatus **9** according to the present invention for monitoring and guiding curettage of a patient's uterus **40.** When using apparatus **9**, which is also shown in Figure 1, endovaginal ultrasound transducer **10** is assembled with connector **12** by inserting transducer **10** into aperture **16** of connector **12**. Cervical holder **14** is then used to grip the cervix **28** of a patient by means of holders **14b**, such that protrusion **18** of connector **12** is locked between arms **14a** when securing the cervical holder. Preferably, endovaginal transducer **10** is then slidably inserted into the fornix of the patient, and its desired orientation is set so as to allow optimal guidance and monitoring of the intra-uterine procedure.

During a uterine procedure, cervical holder **14** and endovaginal ultrasound transducer **10** are preferably held by one hand of the surgeon so that the other hand is free to conduct the surgical procedure. Since the diameter of endovaginal ultrasound transducer **10** is substantially small, the operator may conveniently introduce a medical instrument such as curette **32** through the cervix **28** into the uterine cavity **30** of the patient. The surgical procedure is then carried out and is continuously guided and monitored by means of endovaginal ultrasound transducer **10.** The orientation of transducer **10** relative to connector **12** may be continuously changed as the surgical procedure proceeds.

It will be appreciated by one ordinarily skilled in the art that guiding a curettage is used herein as a non limiting example for guiding any other medical instrument (tool) for diagnostic and/or surgical purposes into the cervix, uterine or fallopian tubes of the patient. Such instruments include, but are not limited to, uterine sound - plastic disposable or stainless steel, uterine dilators - hegar double or single end, uterine curettes, uterine dressing, hysterectomy forceps, ovum forceps, intra-uterine device remover, biopsy punches, endocervical speculum, aspirate curette, vacuum curette, aspirate tube, coagulator, embryo transfer set, insemination device, embryo gamete intra-fallopian transfer (GIFT) catheter, embryo intra uterine insemination (IUI) catheter, Karman™ catheter for uterine aspiration, minimally invasive surgery equipment, such as, grasping forceps, scissors, light dissecting/grasping forceps, diathermy balloon intra cavitary, IUCD, hysterosalpingography catheter, uterine catheter, tubal catheter, brush vacuum intrauterine sound, uterine elevator, Spackmann™ cannula, Scott™ uterine manipulator, Hulka™ controlling tenaculum or forceps, rocket vacuum aspirator curette, uterine depth probe, sampling devices, NOVAK™, KEVORKIAN™, EXPORA™ and Pipelle™.

A common feature to the above listed medical instruments is that they are typically operated by the strong hand (e.g., the right hand of a right handed surgeon) of the surgeon, while apparatus **9** is held and operated by the weak hand (e.g., the left hand of a right handed surgeon) thereof.

According to another embodiment (not shown), connector **12** is used to connect endovaginal ultrasound transducer **10** to an image transmitting device for diagnostic and/or therapeutic purposes such that ultrasound transducer **10** is preferably inserted into the patient's fornix and the image transmitting device is preferably inserted through the cervical canal into the uterine cavity. The image transmitting device may be, for example, an optic fiber, or endoscopy equipment. The image transmitting device may include an image transmitting element such as a CCD or a video camera.

Thus, for example, transducer **10** may be connected by means of connector **12** to an endoscopy equipment so as to allow simultaneous monitoring of the surgical procedure by means of two complementary methods, thereby enabling to accurately determine the position of a medical instrument with relation to the uterine wall.

Apparatus **9** described hereinabove not only allows for ultrasonic view of the treated area in the cervix, uterine or fallopian tube, it further allows for ultrasonic view of the operating medical instrument. This is the case, provided that the medical instrument is brought "inside" or "into" the beam generated by the ultrasound transducer, which beam is shaped as a triangle located within the ultrasound plane of view.

Since apparatus **9** is inserted into a portion of the vagina of the patient prior to the insertion of a medical instrument through the cervix, and further since the medical instrument and apparatus **9** are each held by a different hand of the surgeon, a non-practiced surgeon may find it difficult to bring the medical instrument "inside" or "into" the sonography beam.

As further detailed hereinunder, the following embodiments of the present invention specifically address this problem.

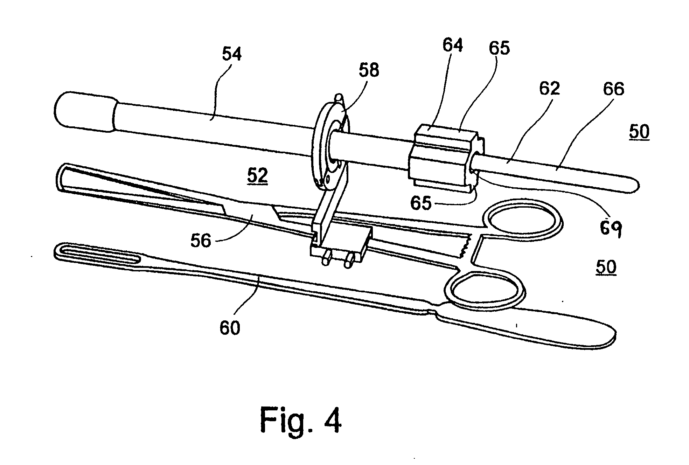

With reference now to Figures 4-7, presented is a system for guidance and monitoring of intra-uterine, cervical and tubal procedures, which is referred to hereinbelow as system **50.**

System **50** includes an assembly **52** typically operable by a weak hand of a surgeon. Assembly **52** includes an endovaginal ultrasound transducer **54** adapted for insertion into a portion of a patient's vagina. Endovaginal ultrasound transducer **54** serves for generating an ultrasound beam. Assembly **50** further includes a cervical holder **56** for holding a patient's cervix. A connector **58** is used for interconnecting ultrasound transducer **54** and cervical holder **56**. Assembly **52**, as so far described, is, in fact, structurally and functionally identical to apparatus **9** (Figure 1) described hereinabove and serves identical proposes. Thus, all the features described hereinabove with respect to apparatus **9** apply also to assembly **52**.

System **50** further includes a medical instrument **60**. Instrument **60** serves to perform the intra-uterine, cervical or tubal procedure and is typically operable by a strong hand of the surgeon. Medical instrument **60** may be a diagnostic instrument, such as, but not limited to, hysterosalpingography catheter, uterine catheter, tubal catheter, brush cytology, cervical adapter for hydrotubation, uterine controlling instruments, vacuum intrauterine sound, uterine elevator, Spackmann™ cannula, Scott™ uterine manipulator, Hulka™ controlling tenaculum or forceps, rocket vacuum aspirator curette, uterine depth probe, sampling devices, NOVAK™, KEVORKIAN™, EXPORA™ and Pipelle™, or a surgical instrument, such as, but not limited to, uterine sound plastic disposable or stainless steel, uterine dilators - hegar double or single end, uterine curettes, uterine dressing, hysterectomy forceps, ovum forceps, intra-uterine device remover, biopsy punches, endocervical speculum, aspirate curette, vacuum curette, aspirate tube, coagulator, embryo transfer set, insemination device, embryo gamete intra-fallopian transfer (GIFT) catheter, embryo intra uterine insemination (IUI) catheter, Karman™ catheter for uterine aspiration, minimally invasive surgery equipment, such as, grasping forceps, scissors, light dissecting/grasping forceps, diathermy balloon intra cavitary and IUCD.

A common feature to the above listed medical instruments is that they are all typically operated by the strong hand (e.g., the right hand of a right handed surgeon) of the surgeon, while assembly **52** is held and operated by the weak hand (e.g., the left hand of a right handed surgeon) thereof.

System **50** further includes a device **62** which serves for monitoring the alignment of medical instrument **60** with respect to endovaginal ultrasound transducer **54** and therefore also with respect to the ultrasound beam generated thereby.

Several exemplary embodiments of device **62** are described hereinbelow. Each of which readily enables the surgeon to align the medical instrument employed with the ultrasound transducer and therefore also with the beam generated thereby. By inserting the medical instrument coaxially with its alignment, the surgeon ensures that the medical instrument is moved on the plane in which the ultrasound beam resides and therefore, eventually the instrument will be visualized in the ultrasound image obtained. This procedure assists the surgeon in bringing the medical instrument "inside" or "into" the ultrasound beam. Device **62** is typically connected to the distal end of transducer **54** via a suitable connector, generally marked as **64**. However, direct connection, and other locations are also envisaged.

Connector **64**, is preferably equipped with wings **65**, being aligned within the plane of the ultrasound beam. To this end, distal end **68** of transducer **54**, is asymmetrically formed, such that when connector **64** is applied thereon, wings **65** acquire their respective positions.

As specifically shown in Figure 4, according to one embodiment, device **62**, includes an extension **66** coaxially connected at a distal end **69** of endovaginal ultrasound transducer **54**, thereby facilitating visual alignment of medical instrument **60** with respect to endovaginal ultrasound transducer **54** and therefore also with respect to the ultrasound beam generated thereby.

According to this embodiment, while inserting medical instrument **60** through the cervix of the patient, the surgeon ensures that instrument **60** parallels extension **66**, to thereby direct instrument **60** "inside" or "into" the ultrasound beam.

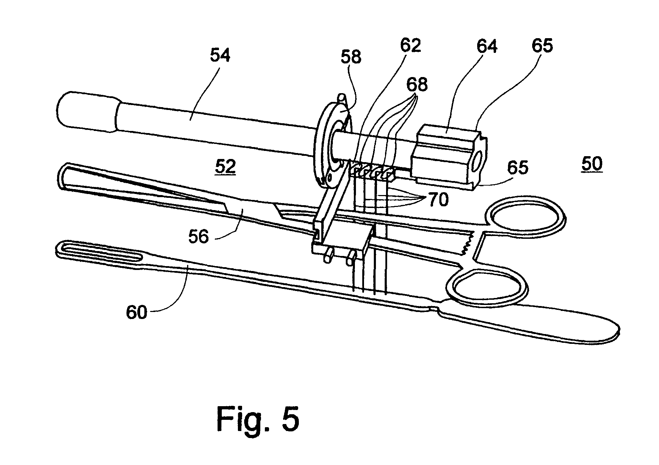

As specifically shown in Figure 5, according to yet another embodiment of the present invention, device **62** includes at least one light beam generator **68** (say, e.g., two, four are shown) connected to assembly **52**, preferably to transducer **54** thereof, preferably via connector **64.** Light beam generators 68 serve for generating at least one focused light beam **70**. Light beams **70** reside substantially in the plane defined by the ultrasound beam of transducer **54**.

When impinge on medical instrument **60**, light beams **70** serve for facilitating visual alignment of medical instrument with respect to endovaginal ultrasound transducer **54** and therefore also with respect to the ultrasound beam.

Each of light beam generators **68** may be, for example, a laser source, generating, for example, a green laser beam, which is known not to be absorbed by living tissues. However, non-coherent light sources are also applicable.

According to this embodiment of the invention, while inserting medical instrument **60** through the cervix of the patient, the surgeon ensures that light beams **70** impinge on instrument **60**, to thereby direct instrument **60** "inside" or "into" the ultrasound beam of transducer **54.** Light beam generators **68** preferably receive energy from a power source, e.g., a battery, implemented in a battery housing located within connector **64**.

Each of generators **68** may be, for example, a pointer type laser diode, having, for example, a maximum output below 5 mW, wavelength of 650 nm, with beam dimensions of about 3.0 nm x 2.5 nm. A suitable diode is the "ES smallest laser pointer" Cat. No. D53,050 which is available from Edmund Scientific, Industrial Optics Division, Barrington, NJ 08007-1380 U.S.A. Generators **68** may alternatively be selected to generate a stripe of light. Edmund Scientific Cat. No. D52,562 "Gamma-x laser light show".

Each of generators **68** preferably further includes a beam splitter, e.g., a TECH SPEC™ pellicle beam splitter. The pellicles are very thin nitrocellulose membranes bonded to lapped aluminium frames. Ghost images are eliminated by the thinness of the membrane as the second surface reflection superimposed on the first surface reflection. The uncoated pellicle reflects 8 % and transmits 92 % through the visible and near infrared regions. The pellicles' thickness is in the range of 2 µm, their index of reflection is (Nd):1.5. Suitable pellicles are available from Edmund Scientific, Industrial Optics Division, Barrington, NJ 08007-1380 U.S.A., Cat. No. D39,478):

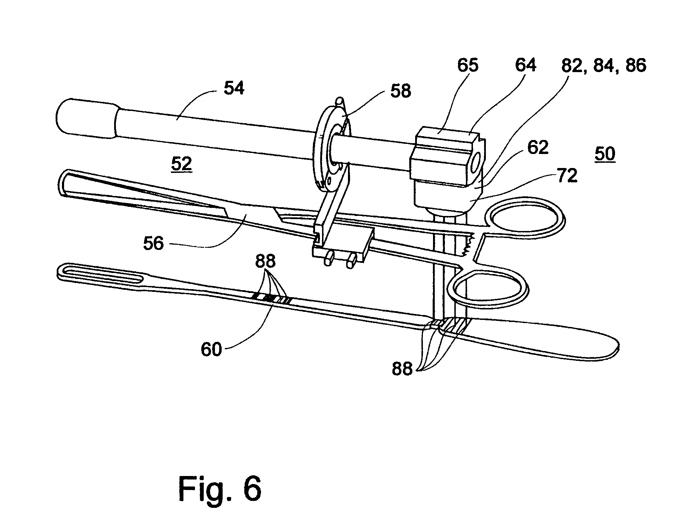

As specifically shown in Figure 6, according to still another embodiment of the present invention, device **62** includes an imaging implement **72** connected to assembly **52**, preferably to transducer **54** thereof, preferably via connector **64**. Imaging implement **72** serves for generating an image of objects in the plane defined by the ultrasound beam. Implement **72** thereby serves for facilitating alignment of medical instrument **60** with respect to endovaginal ultrasound transducer **54** and therefore also with respect to the ultrasound beam generated thereby. According to this embodiment, while inserting medical instrument **60** through the cervix of the patient, the surgeon ensures that imaging implement **72** "sees" or "captures" instrument **60**, to thereby direct instrument **60** "inside" or "into" the ultrasound beam. The image generated by implement **72** is preferably displayed on a screen. A single screen may serve for presenting, in real time, the ultrasound image perceived through transducer **54** and the image perceived through imaging implement **72**.

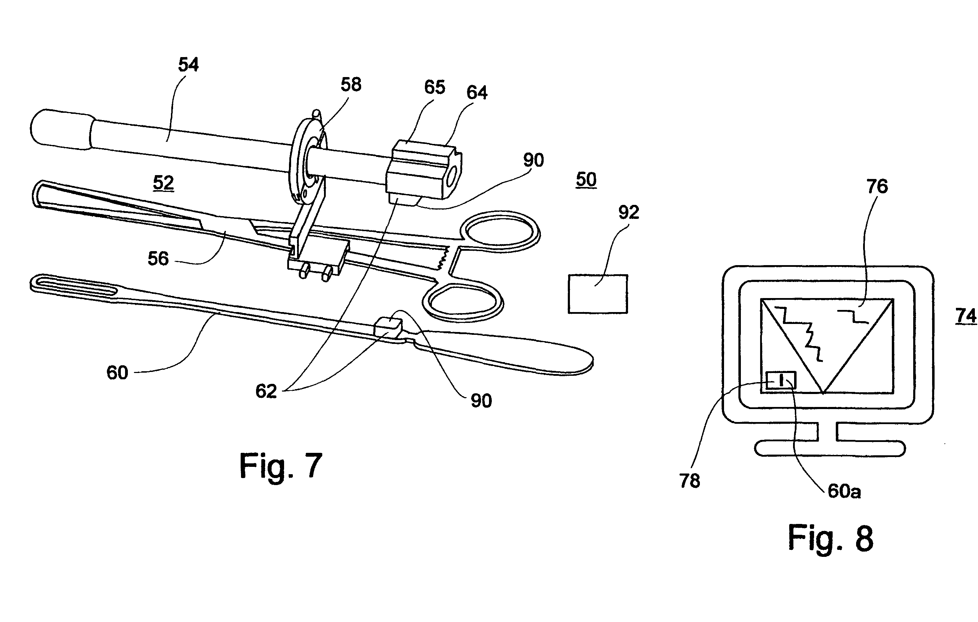

An example is shown in Figure 8, showing a screen **74** displaying an ultrasound image **76** and an image **78**, perceived through implement **72**. Implement **72** is positioned such that when image **78** shows instrument **60** in, for example, a vertical alignment with respect to screen **74**, as indicated in Figure 8 by **60a**, then the surgeon knows that medical instrument **60** is aligned with respect to endovaginal ultrasound transducer **54** and therefore also with respect to the ultrasound beam generated thereby. To this end, screen **74** may further provide a displayed grid or coordinates, such that assessing the verticality of the image **60a** of instrument **60** is facilitated. Implement **72** preferably receive energy from a power source, e.g., a battery, implemented in a battery housing located within connector **64.**

According to a preferred embodiment of the present invention imaging implement **72** is a camera **82**, e.g., a charge coupled device (CCD) camera equipped with a lens or optic fibers arrangement, which is adapted to perceive light in the visible range. According to an alternative embodiment of the present invention implement **72** is a camera sensitive to light in the infrared range, i.e., an infrared (thermal) camera **84**, which may similarly include a lens or an optic fibers arrangement. According to yet another preferred embodiment of the invention imaging implement **72** is an ultrasound implement **86**. According to yet another preferred embodiment of the present invention imaging implement **72** is an X ray implement. In the latter case, an X rays sensitive plate is provided to perceive the image of instrument **60** thereby. Such plates are well known in the art. In the latter case, an X rays

According to each of the imaging embodiments described herein an image of instrument **60** is generated, which image enables the surgeon to direct instrument **60** "inside" or "into" the beam generated by ultrasound transducer **54**.

As further shown in Figure 6, according to a preferred embodiment of the present invention medical instrument **60** is provided with marks **88** along at least a portion thereof. Marks **88** are selected identifiable by imaging implement **72** of choice and are therefore usable for image recognition analysis, which may be used to estimate the depth to which instrument **60** has been inserted at any given time. Image recognition is well known art and therefore will not be further elaborated herein.

The nature of marks **88** must depend on the nature of imaging implement **72** of choice. Thus, if a CCD camera is selected, marks **88** may acquire a color distinguishable from the background color of instrument **60.** If an infrared (thermal) camera is selected, marks 88 may be applied, for example, as substances of increased or decreased heat conductivity as compared with the substance from which instrument **60** is made. If ultrasound or X ray implements are selected, marks **88** may be applied, for example, as holes, recessions, protrusions, etc., to render them distinguishable from the background of instrument **60**. In each of these cases, marks **88** may be further selected distinguishable from one another in a fashion, e.g., similar to a bar-code, such that image recognition analysis may be applied.

A suitable CCD is a CCD sensitive to light at 0.2 lux, having a S/N ratio greater than 46 Db. The CCD is preferably monochromatic and is capable of sensing an area of 6.4 X 4.8 mm. The CCD preferably features miniature size e.g., 30 x 30 x 60 mm, and low weight, e.g., 120 grams. A CCD obeying all of the above criteria is distributed by Edmund Scientific, Cat No. D39,244.

As specifically shown in Figure 7, according to still another embodiment of the present invention device **62** includes at least two electromagnetic field generators **90** which serve for generating electromagnetic fields. One of electromagnetic field generators **90** is connected to assembly **52**, preferably to transducer **54** thereof, preferably via connector **64**. The other electromagnetic field generator **90** is connected to medical instrument **60.** According to this embodiment of the present invention, device **62** further includes at least one electromagnetic field sensor, generally indicated by **92**. Sensor **92** is positioned in a predetermined position in space, such that by analyzing the magnetic fields perceived by electromagnetic sensor **92**, spatial information of the relative locations of electromagnetic field generators **90** and therefore of transducer **54** and medical instrument **60** is obtainable, thereby facilitating alignment of medical instrument **60** with respect to endovaginal ultrasound transducer **54** and therefore also with respect to the ultrasound beam generated thereby. Further description concerning the operation of electromagnetic field generators and electromagnetic field sensors and the use of the latter to retrieve spatial information of the formers is disclosed in, for example, PCT/IL96/00050 (WO 97/03609) and further in U.S. Pat No. 4,945,305. Generators **90** are preferably powered by a mutual power source implemented in a dedicated housing in connector 64 or by independent power sources. Suitable power wiring is envisaged.

A method of guidance and monitoring of intra-uterine, cervical and tubal procedures. is effected by executing the following method steps, in which is a first step a cervical holder is employed for holding a patient's cervix. In a second step an endovaginal ultrasound transducer is inserted into a portion of the patient's vagina. The endovaginal ultrasound transducer serves for generating an ultrasound beam. The ultrasound transducer and the cervical holder are inter-connected there between by a connector to form an assembly which further includes a device for monitoring an alignment of a medical instrument with respect to the endovaginal ultrasound transducer and therefore also with respect to the ultrasound beam generated thereby.

In a third step of the method the procedure is performed by (i) pointing the endovaginal ultrasound transducer such that a treated area is identifiable; and (ii) inserting the medical instrument through the cervix, while holding the patient's cervix by the cervical holder and via the device, aligning the medical instrument with respect to endovaginal ultrasound transducer and therefore also with respect to the ultrasound beam.

In a fourth step the procedure and medical instrument are both monitored in real-time by the ultrasound transducer.

Although the invention has been described in conjunction with specific embodiments thereof, it is evident that many alternatives, modifications and variations will be apparent to those skilled in the art. Accordingly, it is intended to embrace all such alternatives, modifications and variations that fall within the broad scope of the appended claims.

## Claims

1. An apparatus for guidance and monitoring of intra-uterine, cervical and tubal procedures, comprising an assembly (9;52) including:
(a) an endovaginal ultrasound transducer (10; 54) being adapted for insertion into a portion of a patient's vagina;
**characterised in that** the assembly (9;52) further includes:
(b) a cervical holder (14; 56), for holding the patient's cervix; and
(c) a connector (12; 58) for interconnecting said ultrasound transducer (16; 54) and said cervical holder (14; 56).

2. The apparatus of claim 1, wherein said cervical holder (14; 56) includes two arms (14a) having a securing member (14c) and two holders (14b), said holders (14b) being for holding the patient's cervix.

3. The apparatus of claim 1 or 2, wherein said connector (12) includes an aperture (16) for accommodating said ultrasound transducer (10) therein.

4. The apparatus of claim 1, 2 or 3, wherein said connector (12) includes an adjustment member (16a) for adjusting the orientation of said ultrasound transducer (10) relative to said connector (12).

5. The apparatus of claim 2, 3 or 4, wherein said connector (12) includes a protrusion (18) for locking said connector (12) between said arms (14a) of said cervical holder (14) upon securing of said cervical holder (14) by means of said securing member (14c).

6. The apparatus of any preceding claim, wherein said connector (12) and said cervical holder (14) are integrally made.

7. The apparatus of any one of claims 1 to 5, wherein said connector (12) and said ultrasound transducer (10) are integrally made.

8. A system (50) for guidance and monitoring of intra-uterine, cervical and tubal procedures, the system comprising:
(a) an assembly (52) being operable by a weak hand of a surgeon, said assembly (52) being provided by an apparatus according to any preceding claim;
(b) a medical instrument (60) for performing the procedure, said medical instrument being operable by a strong hand of the surgeon; and
(c) a device (62) for monitoring an alignment of said medical instrument (60) with respect to said endovaginal ultrasound transducer (54) of said assembly (52) and therefore also with respect to said ultrasound beam.

9. The system of claim 8, wherein said device (62) includes an extension (66) coaxially connected at a distal end (69) of said endovaginal ultrasound transducer (54) thereby facilitating visual alignment of said medical instrument (60) with respect to said endovaginal ultrasound transducer (54) and therefore also with respect to said ultrasound beam.

10. The system of claim 8, wherein said device (62) includes at least one light beam generator (68) connected to said assembly (52) for generating at least one light beam (70) substantially in a plane defined by said ultrasound beam, when impinges on said medical instrument (60) said at least one light beam serves for facilitating visual alignment of said medical instrument (60) with respect to said endovaginal ultrasound transducer (54) and therefore also with respect to said ultrasound beam.

11. The system of claim 8, wherein said device (62) includes an imaging implement (72) connected to said assembly (52) for generating an image (60a) of objects in a plane defined by said ultrasound beam, thereby facilitating alignment of said medical instrument (60) with respect to said endovaginal ultrasound transducer (54) and therefore also with respect to said ultrasound beam.

12. The system of claim 11, including a screen (74) for displaying said image (60a).

13. The system of claim 11, wherein said imaging implement (72) is a camera (82; 84).

14. The system of claim 13, wherein said camera (82) is sensitive to light in the visible range.

15. The system of claim 13, wherein said camera is an infrared camera (84).

16. The system of claim 11, wherein said imaging implement (72) is an ultrasound implement (86).

17. The system of claim 11, wherein said medical instrument (60) is provided with marks (88) along at least a portion thereof, said marks (88) are identifiable by said imaging implement (72) and are therefore usable for image recognition analysis.

18. The system of claim 8, wherein said device (62) includes at least two electromagnetic field generators (90) for generating electromagnetic fields, one of said electromagnetic field generators (90) is connected to said assembly (52), whereas the other electromagnetic field generator (90) is connected to said medical instrument (60), the device (62) further includes at least one electromagnetic field sensor (92) of a predetermined position, such that by analysing magnetic fields perceived by said at least one electromagnetic sensor (92), spatial information of the relative locations of said electromagnetic field generators (90) and therefore of said assembly (52) and said medical instrument (60) is obtainable, thereby facilitating alignment of said medical instrument (60) with respect to said endovaginal ultrasound transducer (54) and therefore also with respect to said ultrasound beam.

19. The system of claim 8, wherein said medical instrument (60) is selected from the group consisting of an image transmitting device and a surgical instrument.

## Patentansprüche

1. Eine Vorrichtung zum Führen und Überwachen von intrauterinen, Gebärmutterhals- und Eileiter-Behandlungsverfahren, umfassend einen Bausatz (9; 52) bestehend aus:
(a) einem endovaginalen Ultraschallmessfühler (10; 54), der für das Einbringen in einen Teil der Vagina einer Patientin angepasst ist;
**dadurch gekennzeichnet, dass** der Bausatz (9; 52) weiter aus:
(b) einem Gebärmutterhalshalter (14; 56) zum Halten des Gebärmutterhalses der Patientin; und
(c) ein Anschlussstück (12; 58) zum Verbinden des besagten Ultraschallmessfühlers (16; 54) und des besagten Gebärmutterhalshalters (14; 56) besteht.

2. Vorrichtung nach Anspruch 1, wobei der besagte Gebärmutterhalshalter (14; 56) zwei Arme (14a) enthält, die ein Feststellglied (14c) und zwei Halter (14b) aufweisen, wobei die besagten Halter (14b) zum Halten des Gebärmutterhalses der Patientin vorgesehen sind.

3. Vorrichtung nach Anspruch 1 oder 2, wobei das besagte Anschlussstück (12) eine Blende (16) zur Aufnahme des darin befindlichen Ultraschallmessfühlers (10) aufweist.

4. Vorrichtung nach Anspruch 1, 2 oder 3, wobei das besagte Anschlussstück (12) ein Einstellglied (16a) zum Einstellen der Orientierung des besagten Ultraschallmessfühlers (10) relativ zum besagten Anschlussstück (12) aufweist.

5. Vorrichtung nach Anspruch 2, 3 oder 4, wobei das besagte Anschlussstück (12) einen Vorsprung (18) zum Feststellen des besagten Anschlussstücks (12) zwischen den besagten Armen (14a) des besagten Gebärmutterhalshalters (14) nach dem Feststellen des besagten Gebärmutterhalshalters (14) durch die Mittel des besagten Feststellglieds (14c) enthält.

6. Vorrichtung nach einem der vorstehenden Ansprüche, wobei das besagte Anschlussstück (12) und der besagte Gebärmutterhalshalter (14) in einem Stück hergestellt sind.

7. Vorrichtung nach einem der Ansprüche 1 bis 5, wobei das besagte Anschlussstück (12) und der besagte Ultraschallmessfühler (10) in einem Stück hergestellt sind.

8. System (50) zum Führen und Überwachen von intrauterinen, Gebärmutterhals- und Eileiter-Behandlungsverfahren, wobei das System folgendes umfasst:
(a) einen Bausatz (52), der durch die schwache Hand des Chirurgen betätigt wird, wobei der Bausatz (52) durch eine Vorrichtung gemäß einem der vorstehenden Ansprüche bereitgestellt ist;
(b) ein medizinisches Instrument (60) zum Durchführen des Behandlungsverfahrens, wobei das besagte medizinische Behandlungsverfahren durch die starke Hand des Chirurgen betätigt wird; und
(c) ein Gerät (62) zur Überwachung der Ausrichtung des besagten medizinischen Instruments (60) in Bezug auf den besagten endovaginalen Ultraschallmessfühler (54) des besagten Bausatzes (52), und daher ebenfalls in Bezug auf den besagten Ultraschallstrahl.

9. System nach Anspruch 8, wobei das besagte Gerät (62) ein Ansatzstück (66) aufweist, das koaxial am distalen Ende (69) des besagten endovaginalen Ultraschallmessfühlers (54) angeschlossen ist, wodurch das visuelle Ausrichten des besagten medizinischen Instruments (60) in Bezug auf den besagten endovaginalen Ultraschallmessfühler (54), und daher auch in Bezug auf den besagten Ultraschallstrahl ermöglicht wird.

10. System nach Anspruch 8, wobei das besagte Gerät (62) mindestens einen Lichtstrahlerzeuger (68) enthält, der am besagten Bausatz (52) zum Erzeugen von mindestens einem Lichtstrahl (70) auf im Wesentlichen einer Ebene, die durch den besagten Ultraschallstrahl definiert ist, wenn der Aufprall auf das besagte medizinische Instrument (60) von dem besagten mindestens einen Lichtstrahl dem Ermöglichen des visuellen Ausrichtens des besagten medizinischen Instrumentes (60) in Bezug auf den besagten endovaginalen Ultraschallmessfühler (54), und daher auch in Bezug auf den besagten Ultraschallstrahl dient.

11. System nach Anspruch 8, wobei das besagte Gerät (62) eine Bildgebungseinrichtung (72) aufweist, die an dem besagten Bausatz (52) zum Erzeugen einer Abbildung (60a) von Objekten auf einer durch den besagten Ultraschallstrahl definierten Ebene angeschlossen ist, wodurch das Ausrichten des besagten medizinischen Instruments (60) in Bezug auf den besagten endovaginalen Ultraschallmessfühler (54), und daher auch in Bezug auf den besagten Ultraschallstrahl ermöglicht wird.

12. System nach Anspruch 11, enthaltend einen Bildschirm (74) zur Ausgabe der besagten Abbildung (60a).

13. System nach Anspruch 11, wobei die besagte Bildgebungseinrichtung (72) eine Kamera (82; 84) ist.

14. System nach Anspruch 13, wobei die besagte Kamera (82) im sichtbaren Bereich lichtempfindlich ist.

15. System nach Anspruch 13, wobei die besagte Kamera eine Infrarotkamera (84) ist.

16. System nach Anspruch 11, wobei die besagte Bildgebungseinrichtung (72) eine Ultraschalleinrichtung (86) ist.

17. System nach Anspruch 11, wobei das besagte medizinische Instrument (60) mit Markierungen (88) entlang mindestens eines Teils davon vorgesehen ist, wobei die besagten Marierungen (88) durch die besagte Bildgebungseinrichtung (72) identifizierbar sind, und daher für die Bildanalyse geeignet sind.

18. System nach Anspruch 8, wobei das besagte Gerät (62) mindestens zwei Elektromagnetfelderzeuger (90) zum Erzeugen von elektromagnetischen Feldern aufweist, wobei einer der besagten Elektromagnetfelderzeuger (90) an dem besagten Bausatz (52) angeschlossen ist, wobei der andere Elektromagnetfelderzeuger (90) an dem besagten medizinischen Instrument (60) angeschlossen ist, wobei das Gerät (62) weiterhin mindestens einen Elektromagnetfeldsensor (92) von einer vorbestimmten Position enthält, sodass räumliche Informationen der relativen Lagen der besagten Elektromagnetfelderzeuger (90), und dadurch des besagten Bausatzes (52) und des besagten medizinischen Instruments (60) durch Analyse der durch den von dem besagten mindestens einem Elektromagnetfeldsensor (92) wahrgenommenen magnetischen Feldern erreichbar sind, wodurch das Ausrichten des besagten medizinischen Instruments (60) in Bezug auf den besagten endovaginalen Ultraschallmessfühler (54), und daher auch in Bezug auf den besagten Ultraschallstrahl ermöglicht wird.

19. System nach Anspruch 8, wobei das besagte medizinische Instrument (60) aus einer Gruppe ausgewählt wird, bestehend aus einem Bildübertragungsgerät und einem chirurgischen Instrument.

## Revendications

1. Appareil de guidage et de monitorage d'interventions cervicales et tubaires intra-utérines, comprenant un assemblage (9 ; 52) qui inclut :
(a) un capteur d'ultrasons endovaginal (10 ; 54) adapté pour une insertion dans une portion du vagin d'une patiente ;
**caractérisé en ce que** l'assemblage (9;52) comprend en outre:
(b) un support de col (14 ; 56) pour soutenir le col de la patiente ; et
(c) un connecteur (12 ; 58) pour interconnecter ledit capteur d'ultrasons (16 ; 54) et ledit support de col (14 ; 56).

2. Appareil de la revendication 1, dans lequel ledit support de col (14 ;56) comprend deux bras (14a) possédant un élément de fixation (14c) et deux supports (14b), lesdits supports (14b) ayant pour fonction de soutenir le col de la patiente.

3. Appareil de la revendication 1 ou 2, dans lequel ledit connecteur (12) inclut une ouverture (16) dans laquelle ledit capteur d'ultrasons (10) peut être enfermé.

4. Appareil de la revendication 1,2 ou 3, dans lequel ledit connecteur (12) inclut un élément d'ajustement (16a) pour ajuster l'orientation dudit capteur d'ultrasons (10) relativement audit connecteur (12).

5. Appareil de la revendication 2,3 ou 4, dans lequel ledit connecteur (12) inclut une protubérance (18) pour verrouiller ledit connecteur (12) entre lesdits bras (14a) dudit support de col (14) au moyen dudit élément de fixation (14c).

6. Appareil de l'une quelconque des revendications précédentes, dans lequel ledit connecteur (12) et ledit support de col (14) forment un bloc unique.

7. Appareil de l'une quelconque des revendications 1 à 5, dans lequel ledit connecteur (12) et ledit capteur d'ultrasons (10) forment un bloc unique.

8. Système (50) de guidage et de monitorage d'interventions cervicales et tubaires intra-utérines, lequel système comprend:
(a) un assemblage (52) qui est actionné par une main libre du chirurgien, ledit assemblage (52) étant constitué par un appareil selon l'une quelconque des revendications précédentes ;
(b) un instrument médical (60) pour réaliser l'intervention, ledit instrument étant actionné par une main qui opère du chirurgien; et
(c) un dispositif (62) pour monitorer un alignement dudit instrument médical (60) par rapport audit capteur d'ultrasons endovaginal (54) dudit assemblage (52) et, de ce fait, également par rapport au faisceau d'ultrasons.

9. Système de la revendication 8, dans lequel ledit dispositif (62) comprend une extension (66) connectée coaxialement à une extrémité distale (69) dudit capteur d'ultrasons endovaginal (54), facilitant ainsi l'alignement visuel dudit instrument médical (60) par rapport audit capteur d'ultrasons endovaginal (54) et, de ce fait, également par rapport au faisceau d'ultrasons.

10. Système de la revendication 8, dans lequel ledit dispositif (62) comprend au moins un générateur de faisceau lumineux (68) connecté audit assemblage (52) pour générer au moins un faisceau lumineux (70) essentiellement dans un plan défini par ledit faisceau d'ultrasons, lorsqu'on touche audit instrument médical (60) au moins un dit faisceau lumineux sert à faciliter l'alignement visuel dudit instrument médical (60) par rapport audit capteur d'ultrasons endovaginal (54) et, de ce fait, également par rapport au faisceau d'ultrasons.

11. Système de la revendication 8, dans lequel ledit dispositif (62) comprend un dispositif imageur (72) connecté audit assemblage (52) pour générer une image (60a) d'objets dans un plan défini par ledit faisceau lumineux, facilitant ainsi l'alignement dudit instrument médical (60) par rapport audit capteur d'ultrasons endovaginal (54) et, de ce fait, également par rapport au faisceau d'ultrasons.

12. Système de la revendication 11, incluant un écran (74) pour afficher ladite image (60a).

13. Système de la revendication 11, dans lequel ledit dispositif imageur (72) est une caméra (82 ; 84).

14. Système de la revendication 13, dans lequel ladite caméra (82 ; 84) est sensible à la lumière dans le domaine visible.

15. Système de la revendication 13, dans lequel ladite caméra est une caméra infrarouge (84).

16. Système de la revendication 11, dans lequel ledit dispositif imageur (72) est un dispositif à ultrasons (86).

17. Système de la revendication 11, dans lequel ledit instrument médical (60) est pourvu de marques (88) le long d'au moins une portion de ce dernier, lesdites marques (88) étant identifiables par ledit dispositif imageur (72) et étant de ce fait utilisables pour une analyse par reconnaissance d'images.

18. Système de la revendication 8, dans lequel ledit dispositif (62) comprend au moins deux générateurs (90) de champ électromagnétique pour générer des champs électromagnétiques, un desdits générateurs (90) de champ électromagnétique étant connecté audit assemblage (52), alors que l'autre générateur (90) de champ électromagnétique est connecté audit instrument médical (60), le dispositif (62) comprenant en outre au moins un capteur de champ électromagnétique (92) d'une position prédéterminée, de telle sorte qu'en analysant les champs magnétiques perçus par au moins un dit capteur électromagnétique (92), une information spatiale des emplacements relatifs desdits générateurs (90) de champ électromagnétique et, de ce fait, dudit assemblage (52) et dudit instrument médical (60) puisse être obtenue, facilitant ainsi l'alignement dudit instrument médical (60) par rapport audit capteur d'ultrasons endovaginal (54) et, de ce fait, également par rapport au faisceau d'ultrasons.

19. Système de la revendication 8, dans lequel ledit instrument médical (60) est choisi dans le groupe composé d'un dispositif de transmission d'image et d'un instrument chirurgical.
